# EUROPEAN PATENT APPLICATION

(11) **EP 2 269 541 A1**
(43) Date of publication of application: **05.01.2011**
(21) Application number: 09723776.2
(22) Date of filing: 23.03.2009
(51) Int. Cl.: A61F 2/28, A61B 17/56

(54) **BONE CEMENT INJECTING TOOL**

(30) Priority: 24.03.2008 JP 2008075389
(71) Applicant: Terumo Kabushiki Kaisha, Shibuya-ku Tokyo 151-0072 (JP)
(72) Inventor: MATSUMOTO, Atsushi, Ashigarakami-gun Kanagawa 259-0151 (JP)
(74) Representative: TBK-Patent
(86) International application number: PCT/JP2009/055672
(87) International publication number: WO 2009/119509

(57) **Abstract**

[Object]

A bone cement injecting tool capable of preventing the occurrence of air bubbles in the liquid as a pressure transmitting medium.

[Solving Means]

The bone cement injecting tool 10 is composed of a master cylinder 12, a slave cylinder 14, a first gasket 16 that slidably seals the bore of the master cylinder 12, a second gasket 18 that slidably seals the bore of the slave cylinder 14, a tube 20 for communication between the master cylinder 12 and the slave cylinder 14, water 22 filled in the space inside, a piston 24, and a pressure-retaining member 26 that applies a pressure to the forward end of said piston 24. The piston 24 moves the first gasket 16 toward the forward end, thereby delivering the water 22 to the slave cylinder 14 through the tube 20. During storage, the pressure-retaining member 26 pushes the piston 24 toward the fore-end, and this pushing force produces the pressure of the water 22.

## Description

### Technical Field

The present invention relates to a bone cement injecting tool for injection of high-viscosity bone cement into an affected part under X-ray monitoring.

### Background Art

A recent way of dealing with compression fracture of vertebral body induced by cancer and osteoporosis is percutaneous vertebroplasty by injection of a filler which cures with time through a bone biopsy needle inserted into the fractured vertebral body. The filler includes, for example, bone cement based on calcium phosphate capable of forming X-ray images and bone cement based on polymethyl methacrylate (PMMA), which are collectively referred to as bone cement hereinafter. Its injection needs a high pressure of about 3 MPa. The percutaneous vertebroplasty is accomplished by injecting the filler little by little under X-ray monitoring.

The injection of the bone cement employs small diameter syringes ranging from 1 mL to 3 mL in capacity which generate high pressure by manual operation and permit controlled supply in small quantities. The total amount of the bone cement to be injected is about 5 mL. Intermittent injection with small quantities is practiced for adjustment of cure timing.

There has recently been proposed a bone cement injecting tool composed of a biopsy needle and a syringe which are connected to each other with an extension tube. It is capable of extruding the bone cement quantitatively little by little, with a high pressure applied to the syringe (see Patent Documents 1 and 2, for example).

The bone cement injecting tool mentioned above permits extrusion of bone cement little by little comparatively easily without the operator being unduly exposed to X-rays. Unfortunately, it suffers a large pressure loss which results from the high-viscosity bone cement flowing through the thin extension tube. Thus, it needs a large sturdy pressuring component. There has also been proposed a bone cement injecting tool to cope with the foregoing situation. It is constructed such that the syringe to be filled with bone cement is connected to the bone biopsy needle and the syringe is put in action by application of pressure to its gasket through an extension tube by a separate pressuring device (see Patent Documents 3 and 4, for example).

The extension tube for the above-mentioned bone cement injecting tool may have a wire passing through it. In this case there is a large pressure loss due to friction between the wire and the extension tube and this pressure loss has to be compensated for by the pressuring device. This necessitates a strong pressuring device to generate a pressure higher than necessary to push the syringe.

On the other hand, there has been proposed a bone cement injecting tool which employs water in place of a wire for force transmission (see Patent Document 5, for example).

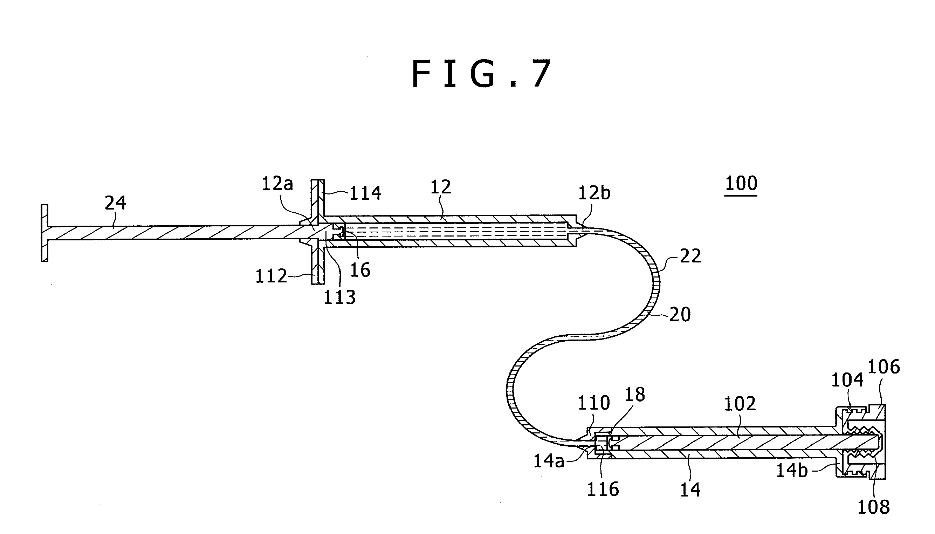

Patent Document 1: US Patent No. 4546767
Patent Document 2: US Patent No. 4671263
Patent Document 3: US Patent Application Publication No. 2005/0070915
Patent Document 4: US Patent Application Publication No. 2005/0113843
Patent Document 5: US Patent Application Publication No. 2007/0027230 (Fig. 7G)

### Disclosure of Invention

### Technical Problem

The bone cement injecting tool disclosed in Patent Document 5 given above has the disadvantage of causing difficulty in secure force transmission through the water passage containing air bubbles compressible under pressure and also causing difficulty in fine quantitative adjustment of bone cement to be injected because the air bubbles expand to apply a residual pressure when the injection of bone cement is temporarily suspended.

It may be possible to remove air bubbles by providing the water passage with a valve; however, this will lead to complicated operation, complex structure, and high cost.

Even though the water passage is free of air bubbles at the time of production, it may become to contain air bubbles after storage for a long period of time.

The present invention was completed in view of the foregoing. It is an object of the present invention to provide a bone cement injecting tool which, during storage, does not allow air bubbles to occur in the liquid as a pressure-transmitting medium, and which, at the time of use, permits the amount of bone cement for injection to be adjusted easily because of the absence of air bubbles.

### Technical Solution

The bone cement injecting tool according to the present invention includes a first cylinder having a first opening at one end and a second opening at the other end, a second cylinder having a third opening at one end and a fourth opening at the other end, said first cylinder being provided with a first seal that slidably seals the bore thereof between said first opening and said second opening, said second cylinder being provided with a second seal that slidably seals the bore thereof between said third opening and said fourth opening, a duct for communication between said second opening and said third opening, a liquid filling the space formed by said first cylinder, said second cylinder, said first seal, said second seal, and said duct, a pressure-retaining member which, during storage, applies a pressure to said liquid through said first seal or said second seal and relieves pressure at the time of use, and a pressing member which moves said first seal toward said second opening and delivers said liquid into said second cylinder through said duct in such a way that the delivered liquid moves said second seal toward said fourth opening, with the resulting movement acting on a prescribed bone cement discharging part, thereby discharging the bone cement.

The bone cement injecting tool mentioned above is constructed such that the pressure-retaining member continues to apply a pressure to the liquid as a pressure-transmitting medium during storage. This construction prevents air bubbles from occurring in the liquid. Moreover, at the time of use, the pressure-retaining member relieves pressure, so that the liquid is free of air bubbles and the amount of bone cement for injection can be easily adjusted. Incidentally, the bone cement broadly denotes any filler capable of curing with time which is used for percutaneous vertebroplasty by injection through a bone biopsy needle inserted into the fractured vertebral body.

Said pressure-retaining member may be easily formed from an elastic body placed between said first cylinder and said pressing member.

Said duct should preferably be a flexible tube so that it facilitates connection with the bone biopsy needle and operation for bone cement injection. Moreover, it properly keeps said first cylinder and said second cylinder away from each other for the operator's radiation protection.

The members constituting the space mentioned above should be formed at least partly from any resin capable of gas diffusion and permeation. Thus, even though gas (air or the like) is present (or dissolved) in the liquid at the time of production, the constituent (resin) permits the gas to diffuse and permeate, so that there exist no bubbles at the time of use.

The above-mentioned structure may be modified such that the bone cement for injection is held in the bore of the second cylinder between the second seal and the fourth opening and the bone cement discharging part includes the fourth opening. This modification simplifies the structure of the bone cement discharging part.

The above-mentioned structure may also be modified such that the second seal is connected to the rod which extends in the axial direction in the bore of the second cylinder, so that the rod acts on the bone cement discharging part. This modified structure permits the bone cement discharging part to be provided with a special part suitable for the technique employed. The resulting bone cement injecting tool will be more versatile.

The above-mentioned structure may also be modified such that the fourth opening is provided with a part for attachment of the bone cement discharging part.

The amount of the above-mentioned liquid should be more than (by 5%) the capacity of the space which is formed when the first seal is positioned at the end of the second opening within the slidable range of the first cylinder and when the second seal is positioned at the end of the third opening within the slidable range of the second cylinder. This modified structure ensures operation and prevents the occurrence of bubbles until more than 5% of water is lost by transpiration through the parts of resin.

The above-mentioned structure may also be modified such that the pressure-retaining member continues to apply a pressure of 10 kPa to 90 kPa to the liquid during storage. This modified structure prevents the entrance of bubbles into the liquid and permits the pressure-retaining member to be handled easily without it becoming too hard.

### Advantageous Effect

The bone cement injecting tool according to the present invention is provided with the pressure-retaining member which continues to apply pressure to the fluid as a pressure transmitting medium during storage. Therefore, it is free from the occurrence of air bubbles in the fluid. Moreover, it permits the amount of bone cement for injection to be controlled easily because there exist no air bubbles in the liquid at the time of use after relieving the pressure by the pressure retaining member. Brief Description of Drawings

[Fig. 1]

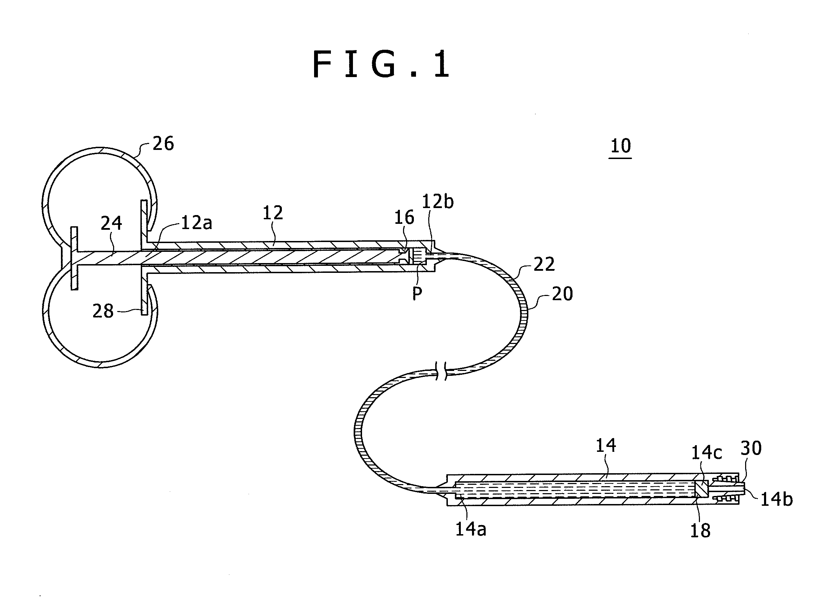

   Fig. 1 is a sectional view showing the bone cement injecting tool according to the first embodiment.
[Fig. 2]

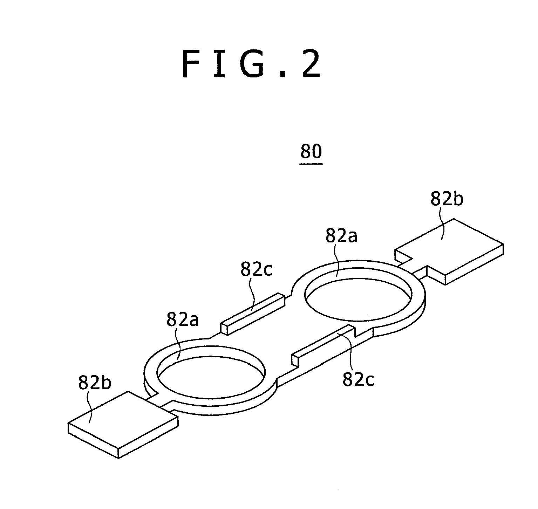

   Fig. 2 is a perspective view showing the rubber band.
[Fig. 3]

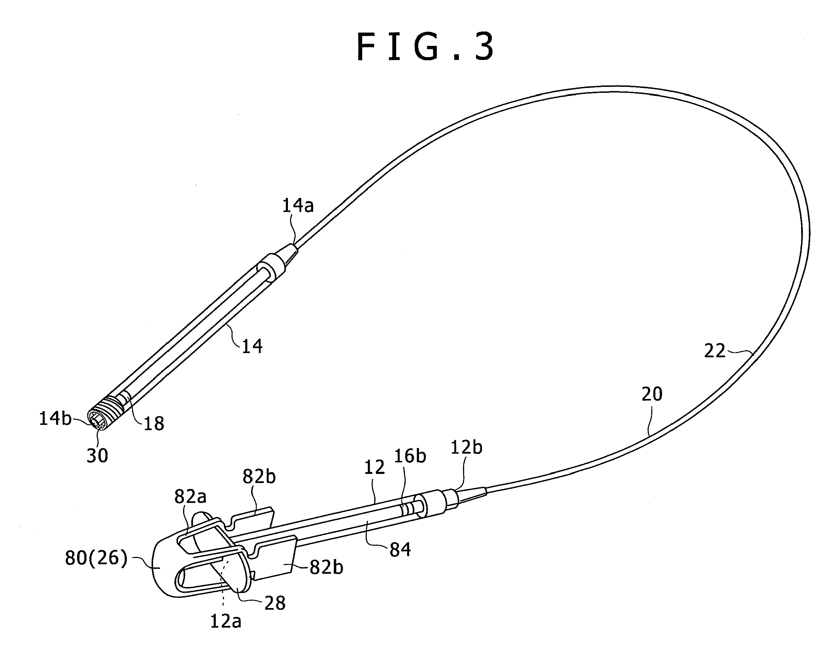

   Fig. 3 is a perspective view showing the bone cement injecting tool with the rubber band attached.
[Fig. 4]

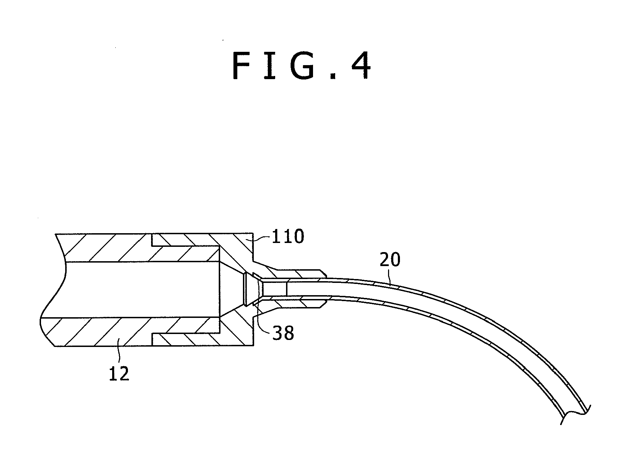

   Fig. 4 is a sectional view showing the swaged structure.
[Fig. 5]

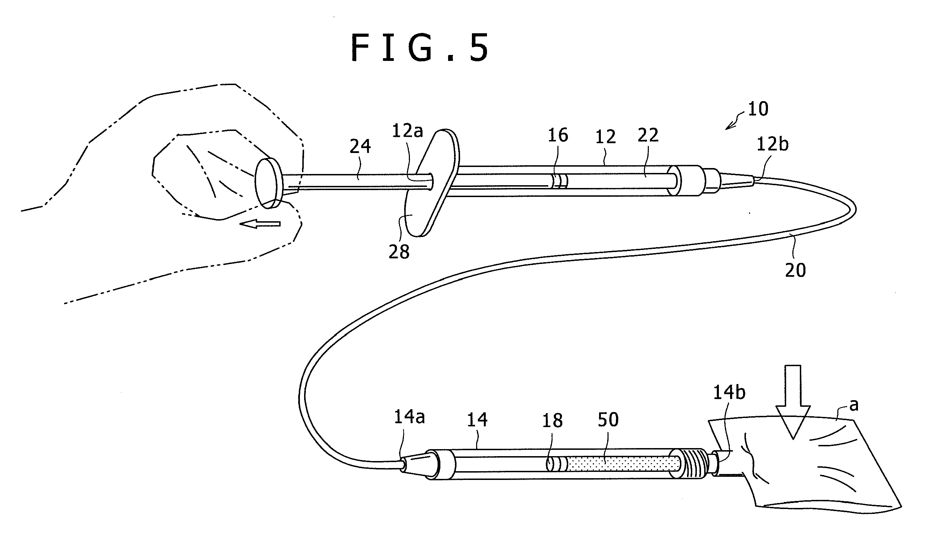

   Fig. 5 is a diagram illustrating how to fill the bone cement through a luer connector by withdrawing the piston.
[Fig. 6]

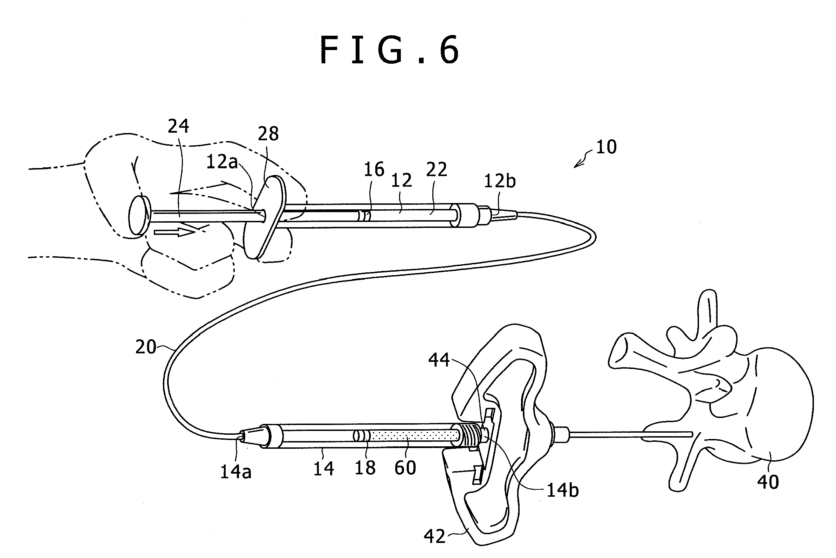

   Fig. 6 is a diagram illustrating the bone cement injecting tool connected to the bone needle.
[Fig. 7]
   Fig. 7 is a sectional view showing the bone cement injecting tool according to the second embodiment.
[Fig. 8]

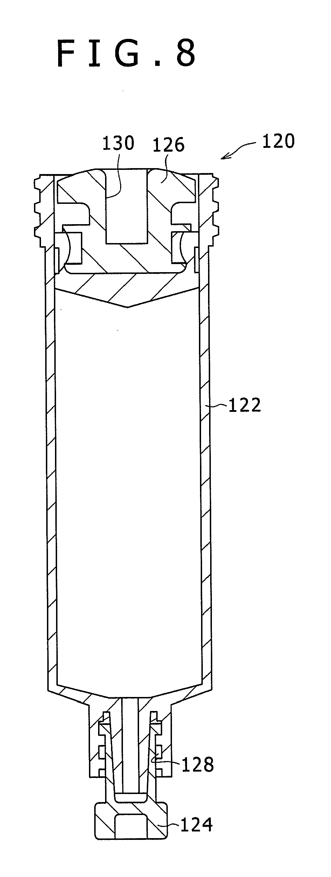

   Fig. 8 is a sectional view of the syringe for injection.
[Fig. 9]

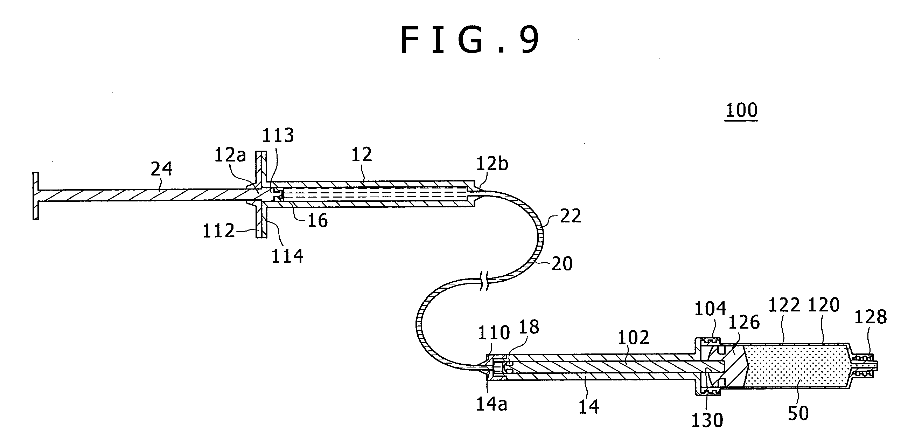

   Fig. 9 is a sectional view of the bone cement injecting tool, with the syringe for injection attached, according to the second embodiment.
[Fig. 10]

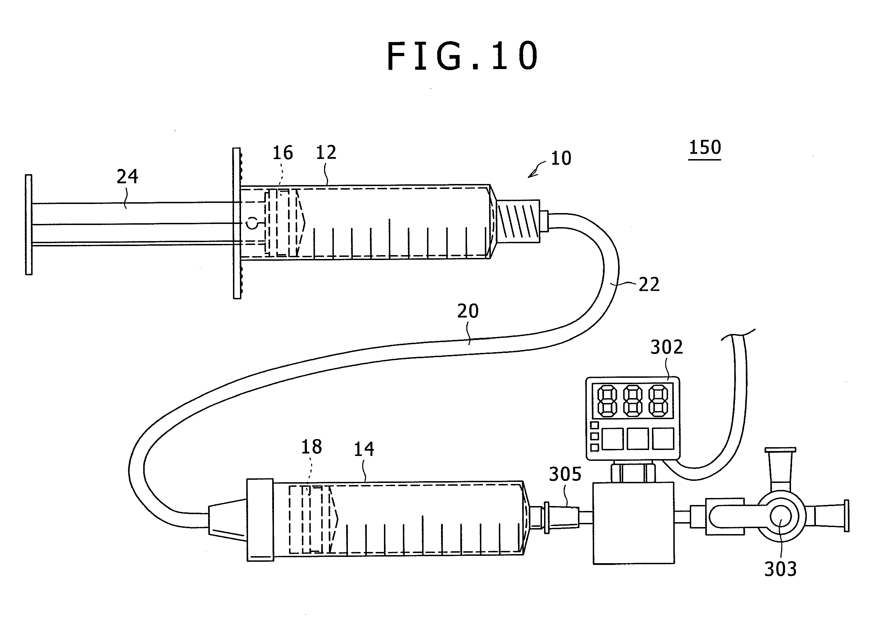

   Fig. 10 is a diagram illustrating the laboratory equipment used in the first experiment example.
[Fig. 11]

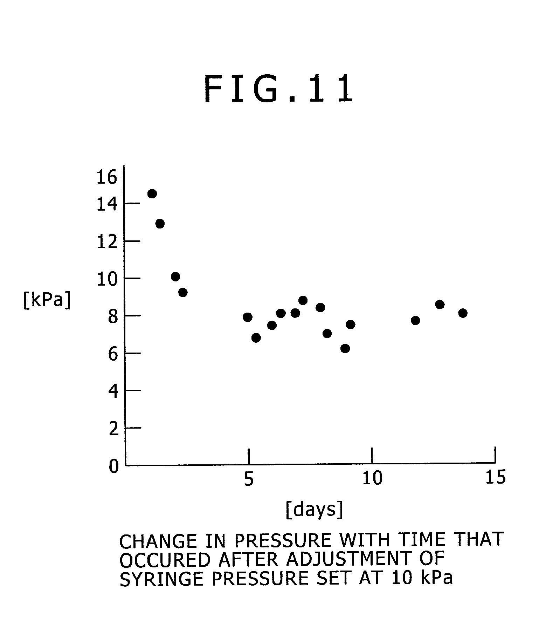

   Fig. 11 is a graph showing the change in pressure with time that occurred after adjustment of syringe pressure.

### Description of Reference Symbols

- 10, 100: Bone cement injecting tool
- 12: Master cylinder (first cylinder)
- 12a: First opening
- 12b: Second opening
- 14: Slave cylinder (second cylinder)
- 14a: Third opening
- 14b: Fourth opening
- 16: First gasket
- 18: Second gasket
- 20: Tube
- 22: Water
- 24: Piston
- 26, 106: Pressure-retaining member
- 30: Luer connector (bone cement discharging part)
- 80: Rubber band (pressure-retaining member)
- 102: Rod
- 120: Syringe for injection (bone cement discharging part)
- 126: Gasket serving as cap

### Best Mode for Carrying Out the Invention

The bone cement injecting tool according to the present invention will be described below with reference to the first and second embodiments and the accompanying drawings Figs. 1 to 11. The bone cement injecting tools 10 and 100 pertaining to the first and second embodiments are intended to deal with compression fracture of vertebral body induced by cancer and osteoporosis with percutaneous vertebroplasty by injection of bone cement which cures with time through a bone biopsy needle inserted into the fractured vertebral body. The bone cement injecting tool 10 pertaining to the first embodiment will be described first.

Fig. 1 shows the bone cement injecting tool 10 in the state of storage pertaining to the first embodiment. The bone cement injecting tool 10 is composed of the master cylinder (the first cylinder) 12, the slave cylinder (the second cylinder) 14, the first gasket (the first seal) 16 which slidably seals the bore of the master cylinder 12, the second gasket (the second seal) 18 which slidably seals the bore of the slave cylinder 14, and the flexible tube (duct) 20 for communication between the master cylinder 12 and the slave cylinder 14. The master cylinder 12 and the slave cylinder 14 are transparent bodies, having graduations on their side wall.

The bone cement injecting tool 10 additionally has the water (liquid) 22 which is filled in the space formed by the master cylinder 12, the slave cylinder 14, the first gasket 16, the second gasket 18, and the tube 20; the piston 24 which moves the first gasket 16 toward the forward end thereby delivering the water 22 to the slave cylinder 14 through the tube 20; and the pressure-retaining member 26 which forces the piston 24 toward the forward end. (In Figs. 1, 7, 9, and 10, the right side represents the forward end and the left side represents the base end.) The water 22 is filled in a state free of gas phase. The pressure-retaining member 26 applies a force which pushes the piston 24 toward the forward end, and this force equals the pressure of the water 22 (or the hydraulic pressure). The liquid filled in the space is preferably water, but may be any aqueous solution such as physiological saline harmless to the living body.

The master cylinder 12 has the first opening 12a which is the opening at the base end and the second opening 12b which is the opening at the forward end. The slave cylinder 14 has the third opening 14a which is the opening at the base end and the fourth opening 14b which is the opening at the forward end. The tube 20 is connected to the second opening 12b and the third opening 14a. The first gasket 16 is slidable between the first opening 12a and the second opening 12b, and the second gasket 18 is slidable between the third opening 14a and the fourth opening 14b. The first gasket 16 is fixed to the forward end of the piston 24. The pressure-retaining member 26 is an elastic body; it continues to apply pressure to the water 22 through the piston 24 while the bone cement injecting tool 10 is in storage. It is removed to relieve pressure when the bone cement injecting tool 10 is in use.

The pressure-retaining member 26 is mounted in such a way that it holds the paired ribs 28 at the base end of the master cylinder 12 and the end of the piston 24. It produces a force to push the piston 24 to the forward end of the master cylinder 12.

The slave cylinder 14 has the luer connector (bone cement discharging part) 30 at its forward end. The luer connector 30 includes the fourth opening 14b and has a simple structure.

The master cylinder 12 and the slave cylinder 14 should be formed at least partly from any resin capable of gas diffusion and permeation. Examples of such resin include polyolefins such as polypropylene, polyethylene, cyclic polyolefin, and polymethylpentene-1, polyester, nylon, polycarbonate, and polyethersulfone. Preferable among these examples is polypropylene, which is almost impermeable to water vapor during sterilization of the water 22 and during long-term storage but is readily permeable to gas. Moreover, polypropylene is inexpensive and has proved itself useful for prefilled syringes. In the case where the tube 20, the first gasket 16, and the second gasket 18 are made of resin, the master cylinder 12 and the slave cylinder 14 may be formed from glass.

During storage, the first gasket 16 in the bore of the master cylinder 12 is placed at the position which is slightly away from the forward end or slightly close to the base end, and it applies pressure to the water 22. The second gasket 18 in the bore of the slave cylinder 14 receives pressure from the water 22, so that it reaches the forward end to which it can slide and it remains in contact with the stepped face 14c.

As the first gasket 16 slides, the master cylinder 12 delivers the water 22 into the tube 20. The maximum amount of the water 22 equals the maximum amount of the bone cement to be discharged from the slave cylinder 14; it is 0.1 mL to 10 mL. The master cylinder 12 and the first gasket 16 are designed such that the empty volume which is formed as the first gasket 16 slides in the master cylinder 12 is larger than the maximum amount plus 5% or up of the bone cement to be discharged. This design ensures the operation of the tool.

That part of the master cylinder 12 along which the first gasket 16 slides has an inside diameter of 2 mm to 14 mm. Also, the master cylinder 12 has an outside diameter of 3 mm to 15 mm.

The slave cylinder 14 and the second gasket 18 are designed such that the empty volume which is formed as the second gasket 18 slides in the slave cylinder 14 is slightly larger than but nearly equal to the maximum amount of the bone cement to be discharged. The empty volume is preferred to be nearly equal to the maximum amount as far as possible. The slave cylinder 14 has an inside diameter which is equal to or larger than that of the master cylinder 12. The slave cylinder 14 has a thickness large enough to withstand the pressure applied to the bone cement and not to be deformed. A thickness larger than 0.6 mm is desirable if the slave cylinder 14 is made of hard polypropylene which is commonly used for disposable syringes.

The first gasket 16 and the second gasket 18 are formed from olefin elastomers, silicone rubber, butyl rubber, fluoro rubber, and other vulcanized rubber. They should preferably be coated with fluorocarbon polymer. The first gasket 16 in the slave cylinder 14 should preferably be formed from butyl rubber or silicone rubber rather than natural rubber in consideration of the fact that it may be attacked or dissolved by methyl methacrylate monomer. Moreover, that part of first gasket 16 which comes into contact with the bone cement containing monomer should preferably be formed by lamination or insert molding from a chemical resistant material such as PEEK and fluorocarbon polymer.

The pressure-retaining member 26 may be formed in any structure from any material so long as it can apply pressure to the water 22 at least after sterilization. The material includes stainless steel spring, plastics such as polypropylene, polycarbonate, and polyurethane, and rubber such as silicone rubber and butyl rubber.

The pressure-retaining member 26 is so constructed as to apply a pressure of 10 kPa to 90 kPa to the water 22 through the piston 24 and the first gasket 16 during storage. The pressure to be applied to the water 22 should be higher than 10 kPa so as to prevent bubbles from occurring in the water 22 during storage. A maximum pressure of about 90 kPa is enough to prevent bubbles from occurring due to temperature change that might occur during manufacture. Moreover, the pressure to be applied to the water 22 should be lower than 90 kPa so as to eliminate the necessity of making the pressure-retaining member 26 excessively hard and to facilitate handling.

As mentioned above, the pressure-retaining member 26 is so constructed as to apply pressure to the water 22 in such a way that the second gasket 18 is in contact with the stepped surface 14c and the first gasket 16 stays anywhere within the sliding range of the master cylinder 12. This structure is desirable because it is simple and inexpensive.

The pressure-retaining member 26 to apply pressure to the water 22 in the bone cement injecting tool 10 may not be restricted to the one mentioned above. It may be any means which is capable of energizing the first gasket 16 toward the second opening 12b or energizing the second gasket 18 toward the third opening 14a, thereby pressurizing the water 22.

A first example of modification of the pressure-retaining member 26 is the rubber band 80 shown in Fig. 2. The rubber band 80 is a sheet-like article having symmetrically arranged two holes 82a, two handgrips 82b at edges, and two projecting parts 82c extending in the widthwise direction at the center. When in use as shown in Fig. 3, the two holes 82a of the rubber band 80 are stretched and hung on the rib 82 of the master cylinder 12, with the center of the rubber band 80 kept in contact with the end of the piston 24. In this way the rubber band 80 applies pressure to the water 22 through the piston 24 and the first gasket 16. The handling of the rubber band 80 is facilitated by grasping the handgrips 82b. The projecting parts 82c prevent the central part of the rubber band 80 from slipping off from the end of the piston 24.

The rubber band 80 may be made of any rubber such as silicone rubber, urethane rubber, and fluoro rubber or any elastomer of olefin type, nylon type, and urethane type. Any existing processing method may be used according to the properties of the material selected. Typical processing methods include press molding, injection molding, and sheet punching.

An example (not shown) of the second modification of the pressure-retaining member 26 may be an elastic rod such as spring inserted through the fourth opening 14b of the slave cylinder 14. In this case, the second gasket 18 is not necessarily positioned at the end of the luer connector 30 side of the slave cylinder 14.

The piston 24 shown in Fig. 1 should be strong enough to withstand force applied by the human hand and resistant to sterilization. It may be made of any of hard polypropylene, polycarbonate, polyarylate, cyclic polyolefin, and metal such as stainless steel.

The tube 20 should preferably be made of comparatively flexible polyolefin, internally or externally reinforced with threads of kevlar, nylon, polyphenylene sulfide, or stainless steel in the form of net or coil, from the standpoint of good pressure resistance. It may also be a multilayered tube having a water-resistant inner layer of polypropylene or fluorocarbon polymer. It should be longer than 5 cm, preferably 15 cm to 50 cm, so that the operator has limited chances of exposure to X-rays at the time of bone cement injection.

The tube 20 should have an inside diameter of 0.1 mm to 2 mm, preferably 0.5 mm to 1 mm. Its outside diameter should be about 1 mm to 3 mm, depending on the wall thickness and the hardness of the resin used.

The tube 20 adequately separates the master cylinder 12 and the slave cylinder 14 from each other, which contributes to improved operability and exposure prevention.

The foregoing parts may be formed by ordinary injection molding, extrusion molding, and press molding. Individual parts may be joined together by adhesion or fusion bonding. Connection of the tube to the cylinders may be accomplished by ordinary method such as swaging shown in Fig. 4 and screw fitting. Swaging shown in Fig. 4 is accomplished by attaching the swaging member 38 to the end of the tube 20, air-tightly inserting the swaging member 38 into the connecting member 110, and ultrasonically bonding the connecting member 110 to the master cylinder. Connection between the tube 20 and the connecting member 110 is not limited and may be accomplished in the usual way by insert molding or integral molding.

The water 22 should be pure water or physiological saline in view of the fact that the molecules in aqueous solution might migrate into the bone cement when the second gasket 18 moves through the slave cylinder 14 containing the bone cement, even though it is not intended for injection into the living body.

The amount of the water 22 may be established as follows. The closed space formed between the first gasket 16 and the second gasket 18 is assumed to have a capacity of 100 when the first gasket 16 is moved to the forward end (indicated by "p" in Fig. 1) of the sliding range and the second gasket 18 is moved to the forward end (as shown in Fig. 1) of the sliding range. Then, the amount of the water 22 should have a volume larger than 105. In this way it is possible to securely move the second gasket 18 in the slave cylinder 14 up to the forward end of the sliding range. The amount of the water 22 should be sufficient to compensate for loss of water due to transpiration through the tube wall made of resin. This prevents shortage of the aqueous solution and occurrence of bubbles during storage.

Otherwise, the amount of the water 22 will decrease due to transpiration of the aqueous solution inside during storage and distribution, and the decrease of the water causes the first gasket 16 to move to the limit of the sliding range in the master cylinder 12, thereby preventing the situation in which the water 22 does not apply force any longer. The rate of transpiration of the water depends on the area of individual members in contact with the water 22 and the water vapor permeability of individual members. The water vapor permeability also depends on the thickness of each of the members in contact with the water 22. For example, a polypropylene tube should have an average thickness greater than 0.6 mm.

The maximum amount of the water 22 should be determined according to the capacity of the master cylinder 12 and the slave cylinder 14 and the design value of the bone cement to be discharged from the slave cylinder 14. It should preferably be equal to or smaller than 300 if the capacity of the closed space mentioned above is 100.

The water 22 is filled into the bone cement injecting tool 10 in the following manner. The second gasket 18 is fitted into the forward end of the slave cylinder 14, and the tube 20 with master cylinder 12 is fusion-bonded to the slave cylinder 14. A pipe capable of passing through the tube 20 of the slave cylinder 14 is inserted and the water 22 is filled into the master cylinder 12, the slave cylinder 14, and the tube 20. Suction is applied to the end of the master cylinder 12 or a pneumatic pressure is applied through the luer connector 30, so that the second gasket is moved toward the base end. The first gasket 16 and the piston 24 are pushed in through the opening of the master cylinder 12 in such a way as to avoid entrance of air bubbles. Finally, the pressure-retaining member 26 is put on to hold the piston 24 and the flange 114. The pressure applied to the water 22 should be previously designed from the inside diameter of the master cylinder 12 and the stress applied by the deformation of the pressure-retaining member 26, so that the above-mentioned pressure is produced.

The bone cement injecting tool 10 is sterilized by autoclaving or electron radiation and then packaged and stored, with the water 22 kept pressurized. Storage also includes the period for distribution for marketing.

The following deals with the function of the bone cement injecting tool 10 constructed as mentioned above.

During storage, the pressure-retaining member 26 keeps the water 22 pressurized, and hence there is no possibility of gas entering the water 22. Even if a few air bubbles enter the water 22 during manufacture, they will diffuse and permeate through the resin part by the action of the pressure-retaining member 26 and no gas remains at the time of use.

At the time of use, the pressure-retaining member 26 is removed from the piston 24 and the master cylinder 12 so as to relieve the pressure applied to the water 22. The bone cement 50 (see Fig. 5), which has been prepared from a powder agent and a monomer agent by mixing together, is placed in another container "a."

The luer connector is connected to the container as shown in Fig. 5 and the container is compressed so that the high-viscosity bone cement 50 is poured into the slave cylinder 14. This procedure should be completed before the bone cement 50 cures. The container "a" may be a syringe which is connected to the slave cylinder through a three-way stopcock.

The bone cement 50 may be cooled in an ice bath to control the curing rate. During cooling, if there exist air bubbles in the water 22, the bone cement 50 which has been once filled into the slave cylinder will be pushed out by the force of expanding air bubbles. However, the bone cement 50 will not leak from the forward end because the water 22 does not contain air bubbles in the case of the bone cement injecting tool 10, ensuring good operability.

The luer connector 30 of the slave cylinder 14 is connected to the female connector 44 of the bone needle 42 resembling a hollow pipe which has been inserted into the vertebra 40 at the affected part.

The piston 24 of the master cylinder 12 is pressed, so that the first gasket 16 applies pressure to the water 22 and the pressure is transmitted to the second gasket 18. As the first gasket 16 moves to the forward end of the master cylinder 12, and the second gasket 18 also moves to the forward end of the slave cylinder 14, because the water 22 is a substantially incompressible fluid, thereby injecting the bone cement 50 into the vertebra 40 through the luer connector 30 and the bone needle 42. Although this procedure is usually carried out under X-ray monitoring, the operator is almost free of radiation exposure because he operates the master cylinder 12 away from the affected part and the slave cylinder 14.

If there exist air bubbles in the water 22, they become compressed and prevent the bone cement 50 from being discharged constantly in proportion to the movement of the piston 24. This is because the bone cement 50 is highly viscous and needs a high pressure (about 3 MPa at the maximum) for injection into the bone. The high injection pressure compresses most of air bubbles due to the atmospheric pressure but does not inject the bone cement 50 even though the piston 24 advances. Even though the action of the piston 24 is suspended or reduced on the way, the pressure due to compressed air bubbles remains and expands the air bubbles to discharge the bone cement 50 wastefully. This prevents the bone cement 50 from being discharged quantitatively.

By contrast, the bone cement injecting tool 10 (and 100) according to this embodiment eliminates the chance of air bubbles entering the water 22. Therefore, it does not involve bubble compression at the time of highpressure injection and hence it permits the bone cement 50 to be discharged constantly in response to the movement of the piston 24. Moreover, when the action of the piston 24 is suspended or reduced on the way, the delivery of the bone cement 50 also suspends or reduces in response to the movement of the piston 24 because there is no gas expansion. This makes it easy to control the amount of injection of the bone cement 50. In addition, the amount of the bone cement 50 which has been actually injected can be known from the gradations on the master cylinder 12 and the amount of movement of the first gasket 16.

After an adequate amount of the bone cement 50 has been injected into the affected part, the bone needle 42 is removed to complete the necessary procedure.

The following is a description of the bone cement injecting tool 100 according to the second embodiment. Those parts common to the bone cement injecting tools 10 and 100 are indicated by the same reference numerals and their detailed description is omitted.

Fig. 7 shows the bone cement injecting tool 100 in the state of storage according to the second embodiment. The bone cement injecting tool 100 is composed of the master cylinder 12, the slave cylinder 14, the first gasket 16, the second gasket 18, the tube 20, the water 22, and the piston 24.

The bone cement injecting tool 100 is further composed of the rod 102 which extends in the slave cylinder 14 and has the second gasket 18 attached to its base end side, the skirt 104 attached to the forward end of the slave cylinder 14, and the pressure-retaining member 106 attached to the skirt 104. The skirt 104 may be provided with the syringe 120 for injection which will be mentioned later.

The pressure-retaining member 106 corresponds to the pressure-retaining member 26 mentioned above. It is removably fitted to the skirt 104, and it also has the elastic part 108 resembling bellows inside thereof. The elastic part 108 is in contact with the forward end of the rod 102, so that it pushes the rod 102 toward the base end. Alternatively, the pressure-retaining member 106 may be screwed up to the skirt 104.

The second gasket 18 is attached to the rod 102 at the base end side, so that it prevents the water 22 from leaking into the slave cylinder 14. The rod 102 is made of the same hard material as the piston 24, and it is connected to the second gasket 18 by fitting, insert molding or the like.

The slave cylinder 14 and the tube 20 are air-tightly joined together through the connecting member 110. In other words, as in the structure shown in Fig. 1, the swaging member 38 is attached to the end of the tube 20, air-tightly inserted into the connecting member 110, and then ultrasonically bonded to the master cylinder. The tube 20 and the connecting member 110 may be integrally joined together by injection molding, with the former inserted into the latter, and the connecting member 110 may be fusion-bonded to the slave cylinder 14 at the time of assembling. It is also possible to achieve the foregoing object by inserting the tube 20 into the slave cylinder 14 at the time of its injection molding.

The master cylinder 12 has, at the base end side, the round flange 114 corresponding to the above-mentioned rib 28. The flange 114 has the stopper 112 fusion-bonded thereto. The piston 24 is inserted into the hole of the stopper 112. This structure is intended to apply pressure to the water 22.

The piston 24 has the large-diameter part 113 near the part connected to the first gasket 16. This large-diameter part 113 does not pass through the hole of the stopper 112. Therefore, the first gasket 16 does not slip off from the master cylinder 12 even when pressure is applied to the water 22.

To fill the water 22, the fourth opening 14b of the slave cylinder 14 with the second gasket 18 not attached is dipped in an aqueous solution placed in a water bath, and the piston 24 is reciprocated to repeat sucking and discharging until air bubbles disappear. With the piston 24 positioned at the fore-end side of the master cylinder 12, the second gasket 18 is inserted through the fourth opening 14b of the slave cylinder 14, with care to avoid entrance of air bubbles.

Then, the rod 102 and the second gasket 18 are inserted into the slave cylinder 14 to such an extent that the piston 24 comes into contact with the stopper 112 and the first gasket 16 does not move any more. Then, the pressure-retaining member 106 is screwed up to the fourth opening 14b.

In the foregoing state, as shown in Fig. 7, there should remain the excess aqueous solution 116 in that part of the slave cylinder 14 where the second gasket 18 can slide. The total amount of the water 22 that includes the part of the excess aqueous solution 116 should preferably be 105 to 300 if the volume of the space fluid-tightly closed by the first gasket 16 and the second gasket 18 is assumed to be 100, with the piston 24 pulled out to the limit in the master cylinder 12 and the second gasket 18 positioned closest to the base end in the slave cylinder 14.

The bone cement injecting tool 100 may be filled with the bone cement 50 by using the injecting syringe (bone cement discharging part) 120 shown in Fig. 8, which can also be used as a container when the bone cement 50 is prepared.

The injecting syringe 120 is composed of the housing 122, the fore-end cap 124, and the gasket 126 which serves also as the cap. The housing 122 is constructed such that its rear end can be connected to the skirt 104 of the bone cement injecting tool 100. In addition, the housing 122 has, at its fore-end, the luer fitting part 128 with locking to which the bone needle 42 (shown in Fig. 6) can be connected.

The bone cement is prepared (by mixing two components) in the injecting syringe 120. Alternatively, the bone cement which has been prepared is poured into the injecting syringe 120 and then the gasket 126 which also serves as the cap is connected to it. After that, the pressure-retaining member 26 is removed and the injecting syringe 120 is connected in such a way that the end of the rod 102 comes into contact with the rodinserting hole 130 of the gasket 126 which also serves as the cap.

Pushing the piston 24 exerts pressure on the water 22 in the master cylinder 12, and this pressure propagates to the second gasket 18 to move forward the rod 102 and the gasket 126 which serves also as the cap, thereby discharging the bone cement 50 from the injecting syringe 120. The bone cement can be injected into the living body through the living body access device such as the bone needle 42 or the like attached to the luer fitting part 128.

The bone cement injecting tool 100 according to the second embodiment is versatile because it permits one to use the injecting syringe 120 which is specially designed as the bone cement discharging part that suits the specific technique selected. Moreover, the fact that the bone cement is stored in the injecting syringe 120 eliminates the step for placing the bone cement which has been prepared in a separate container into the slave cylinder 14.

The bone cement injecting tools 10 and 100 according to the embodiments mentioned above employ the pressure-retaining member 26 or 106 to apply pressure to the water 22 as the pressuring transmitting medium during storage. This system prevents the occurrence of air bubbles in the water 22. Moreover, when the pressure-retaining member 26 or 106 relieves pressure, the water 22 is free of air bubbles at the time of use, and this facilitates controlling the amount of the bone cement to be injected.

The function of the bone cement injecting tool 10 was experimentally examined. The results of the experiment are explained in the following.

The first experiment was carried out by using the master cylinder 12 and the slave cylinder 14, both of which are commercial 1-mL syringe of polycarbonate having an inside diameter of 5 mm and a wall thickness of 2.5 mm, the tube, which is a nylon tube with stainless steel braid having an inside diameter of 1 mm, an outside diameter of 1.5 mm, an internal coating layer of fluorocarbon polymer , and the first gasket 16 and the second gasket 18, both of which are made of silicone. The amount of the bone cement injected into the tool was 1 mL. The master cylinder 12 was connected to the tube 20 by means of a luer, so that connection was accomplished after water filling. The slave cylinder 14 was connected to the tube by metal swaging to ensure fluid-tightness. The water 22 was distilled water which has previously been stored in a refrigerator. This distilled water gave off air bubbles while it was allowed to stand at room temperature under the atmospheric pressure.

After the water 22 had been filled into the bone cement injecting tool 10, instead of the pressure-retaining member 26, a rubber band was put around the rib 28 and the rear end of the piston 24, so that it exerts compressing force. The space close to the luer of the slave cylinder 14 was filled with the water 22, and the pressure of the water 22 was adjusted to 10 kPa by means of the rubber band.

The bone cement injecting tool 10 prepared in the foregoing manner gave off a few air bubble one hour after assembling; however, the air bubbles disappeared on standing at room temperature for one week or more. No air bubbles appeared after that during storage. The slave cylinder 14 was filled with commercial bone cement (Osteobond from Cook Corp.). The bone cement 50 was smoothly discharged in response to the movement of the piston 24 as the piston 24 was pushed.

For comparison with the first experiment, the bone cement injecting tool 10 of the same structure as above was allowed to stand without rubber band attached. After one hour, the occurrence of air bubbles was noticed in the water 22 in the slave cylinder 14. After that the slave cylinder 14 was filled with the bone cement. As the piston 24 was slightly pushed, the bone cement discharged little by little from the luer connector. Even though the piston 24 was stopped, the bone cement continued to discharging. In other words, the amount of the bone cement discharged was not in direct proportion to the movement of the piston 24. Moreover, the number of air bubbles increased according as the tool was allowed to stand at room temperature.

The second experiment was carried out in the following manner. The bone cement injecting tool 10 was similar to that used in the first experiment except that the cylinder was a 5-mL syringe covered with an outer cylinder of cyclic polyolefin which is commercially used for prefilled syringes. The cylinder was filled with refrigerated water and the tool was allowed to stand for one hour in an oven at 50°C, with a pressure of 25 kPa applied. The first gasket 16 and the second gasket 18 were those of butyl rubber. The pressure of the water 22 rose up to 88.7 kPa, but no air bubbles were noticed.

The third experiment will be explained in the following.

The occurrence of air bubbles is due to the fact that gas in the atmosphere dissolves in water through the material for the components and the dissolved gas separates out in the form of bubbles in response to the change in room temperature which changes the solubility of dissolved gas. Therefore, the air bubbles will not appear if a pressure is applied which is high enough to suppress air bubbles that occur due to temperature change during storage. To prove that air bubbles occurring in the syringe disappear, the third experiment was carried out with the apparatus 150 shown in Fig. 10.

The third experiment employed a commercial 10-mL syringe of polypropylene as the slave cylinder 14. The bone cement injecting tool 10 was filled with water, which had been cooled and stored in a refrigerator, in such a way that no gas entered. The pressure gage 302 was connected through the luer connector 305. The pressure in the slave cylinder 14 was adjusted by pouring water through the three-way pressure-adjusting port 303. At this point of time, there existed no gas at all in the bone cement injecting tool 10, the slave cylinder 14, and the pressure gage 302.

The bone cement injecting tool 10 for experimental use was placed in an oven at 50°C for one hour and then the pressure was adjusted to the prescribed value. After that, the pressure was measured at least once a day, and the tool was stored for a long period of time, during which the pressure was adjusted. The results of the experiment are shown in Table 1.

**[Table 1]**

| Change with time of bubbles at 50°C | | | | |
|---|---|---|---|---|
| Set pressure | At the time of adjustment after storage at 50°C | | | Disappearance of bubbles |
| [kPa] | Max. pressure | Min. pressure | Average ± S.D. | |
| | [kPa] | [kPa] | [kPa] | |
| 0 | 9.6 | 1.3 | 4.0 ± 2.3 | Not disappeared for 2 weeks |
| 10 | 14.4 | 2.5 | 8.4 ± 2.6 | Not disappeared for 2 weeks |
| 20 | 20.5 | 10 | 16.0 ± 2.6 | Disappeared within 8 days |
| 27 | 23.3 | 22.7 | 23.0 ± 0.4 | Disappeared within 2 days |
| 30 | 26.5 | 14.8 | 20.8 ± 5.7 | Disappeared within 2 days |

It was found that air bubbles in the master cylinder 12 and the slave cylinder 14 disappear when a pressure greater than 20 kPa is continuously applied. The rate of disappearance increases in proportion to the pressure, and bubbles disappeared within about two days.

Even though the pressure is set at 20 kPa, the pressure gradually decreases with time. The experimental apparatus shown in Fig. 10 used in the first experiment permits one to observe the occurrence and disappearance of air bubbles sensitively because it has the master cylinder 12 and the slave cylinder 14 which remain almost constant in volume. Therefore, the pressure decrease is attributable to the release of gas from water in the master cylinder 12 and the slave cylinder 14. On the other hand, in the case where no pressure is applied, the pressure somewhat increases due to the occurrence of air bubbles, but the pressure increase to such an extent does not cause air bubbles to disappear. That is, so long as the internal pressure is about 10 kPa in both the cylinders (the master cylinder 12 and the slave cylinder 14), gas molecules have a weaker tendency to diffuse outward than inward and hence air bubbles do not disappear. By contrast, if the pressure is raised to 20 kPa, gas diffuses from both of the cylinders into the atmosphere; that is, outward gas diffusion becomes predominant. There will be a condition under which water in both of the cylinders does not diffuse into the atmosphere or only such gas components as oxygen and nitrogen undergo exchange through the resin walls. Under such a condition, the pressure in the cylinders remains almost constant for a certain period of time and gas components diffuse inward and outward equally.

In the case where a pressure of 10 kPa is applied to the water, the pressure at the time of adjustment is as shown in Fig. 11. It is noted that immediately after the start of the test, the pressure is higher than 10 kPa at the time of adjustment owing to the occurrence of air bubbles, but several days later, the pressure becomes almost constant about 8 kPa close to the set pressure. This suggests that the pressure of about 8 kPa to 10 kPa is that at which the outward gas diffusion balances with the inward gas diffusion in both of the cylinders.

However, even though polypropylene is characterized by its low water vapor permeability, it does not necessarily have a completely null water vapor permeability. In other words, the volume of water in both of the cylinders decreases with time and the partial pressure in them also tends to decrease in proportion to the decreased volume of water. The result is that the pressure at which the gas in both of the cylinders diffuses inward and outward at the same rate will be a higher one in the case of a resin which is completely impermeable to water vapor.

In view of the fact that the inside of each air bubble is filled with saturated water vapor, it is necessary to subtract the partial pressure of saturated water vapor from the internal pressure of the cylinder in order to obtain the partial pressure of gas components such as oxygen and nitrogen inside air bubbles. For the incomings and outgoings of oxygen and nitrogen molecules passing through the resin separating the inside and outside of the cylinder to become balanced, it is necessary that the partial pressure of gas is equal inside and outside the cylinder. This implies that the partial pressure of oxygen gas and nitrogen gas becomes balanced inside and outside if the water inside is pressurized as much as the pressure of saturated water vapor. In fact, the pressure of saturated water vapor is 12.3 kPa at 50°C, and this satisfies the result shown in Table 3. The foregoing suggests that if the bone cement injecting tool according to the present invention is stored and distributed below room temperature, it will be possible to prevent the occurrence of air bubbles and remove air bubbles at a lower pressure in the cylinders because the pressure of saturated water vapor is 2.3 kPa at room temperature (20°C). However, in consideration of the fact that the bone cement injecting tool would temporarily experience a high temperature during distribution, it is desirable to keep the cylinder internal pressure above 10 kPa, which is a condition under which air bubbles do not occur and air bubbles can be eliminated at about 50°C.

The period of two days which is required for the disappearance of air bubbles is sufficiently shorter than the time required for acquisition of lot control data for quality assurance after production and for distribution. The foregoing experiment was started under the condition for air bubbles to occur very easily. It is apparent that there will be no possibility of air bubbles appearing during storage and distribution if the bone cement is filled after deaeration or in the absence of air bubbles.

The bone cement injecting tool according to the present invention is not limited to the one illustrated in the foregoing embodiments but it may be changed and modified within the scope of the present invention.

## Claims

1. A bone cement injecting tool comprising:
a first cylinder having a first opening at one end and a second opening at the other end;
a second cylinder having a third opening at one end and a fourth opening at the other end;
said first cylinder being provided with a first seal that slidably seals the bore thereof between said first opening and said second opening;
said second cylinder being provided with a second seal that slidably seals the bore thereof between said third opening and said fourth opening;
a duct for communication between said second opening and said third opening;
a liquid filling the space formed by said first cylinder, said second cylinder, said first seal, said second seal, and said duct;
a pressure-retaining member which, during storage, applies a pressure to said liquid through said first seal or said second seal and relieves pressure at the time of use; and
a pressing member which moves said first seal toward said second opening and delivers said liquid into said second cylinder through said duct, wherein
the delivered liquid moves said second seal toward said fourth opening, with the resulting movement acting on a prescribed bone cement discharging part, thereby discharging the bone cement.

2. The bone cement injecting tool as defined in Claim 1, wherein said pressure-retaining member is an elastic body placed between said first cylinder and said pressing member.

3. The bone cement injecting tool as defined in Claim 1 or 2, wherein said duct is a flexible tube.

4. The bone cement injecting tool as defined in any one of Claims 1 to 3, wherein at least part of the member constituting said space is formed from a resin capable of gas diffusion and transmission.

5. The bone cement injecting tool as defined in any one of Claims 1 to 4, wherein
the bore of said second cylinder holds the bone cement for injection between said second seal and said fourth opening, and
said bone cement discharging part includes said fourth opening.

6. The bone cement injecting tool as defined in any one of Claims 1 to 5, wherein said second seal is connected to the rod which extends in the axial direction in the bore of said second cylinder and said rod acts on said bone cement discharging part.

7. The bone cement injecting tool as defined in Claim 6, wherein said fourth opening is provided with a mounting portion for mounting said bone cement discharging part.

8. The bone cement injecting tool as defined in any one of Claims 1 to 7, wherein
the amount of said liquid is larger by 5% or up than the design capacity of said space which is defined when said first seal is positioned at the end of said second opening side within the slidable range of said first cylinder and said second seal is positioned at the end of said third opening side within the slidable range of said second cylinder.

9. The bone cement injecting tool as defined in any one of Claims 1 to 8, wherein said pressure-retaining member applies a pressure of 10 kPa to 90 kPa to said liquid during storage.
